# EUROPEAN PATENT APPLICATION

(11) **EP 2 514 421 A1**
(43) Date of publication of application: **24.10.2012**
(21) Application number: 12164215.1
(22) Date of filing: 16.04.2012
(51) Int. Cl.: A61K 31/44, A61P 9/02

(54) **Composition comprising torasemide**

(30) Priority: 18.04.2011 NL 2006622; 22.07.2011 US 201161510548 P
(71) Applicant: Eurovet Animal Health B.V., 5531 AE Bladel (NL)
(72) Inventor: Boeren, Marinus Maria Martinus, 5063 EC OISTERWIJK (NL); Vermeer, Johanna Elisabeth Maria, 5091 TA MIDDELBEERS (NL); Michiels, Henricus Franciscus Catharina, 5541 EM REUSEL (NL)
(74) Representative: Algemeen Octrooi- en Merkenbureau

(57) **Abstract**

The present invention relates to the alleviation or treatment of one or more symptons caused by one or more conditions or diseases selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof. The present invention further relates to a kit and a method for alleviating or treating said symptoms.

## Description

The present invention relates to a composition for the alleviation or treatment of one or more symptoms caused by one or more conditions or diseases. The present invention further relates to a kit and a method for alleviating or treating said symptoms.

Torasemide (also known as torsemide) is a loop diuretic and is for example disclosed in DE2516025 and US 4018929. These documents also disclose the diuretic properties of torasemide in rats. Torasemide has the following formula:

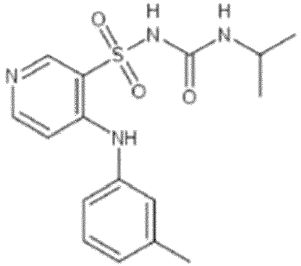

Further, diuretic properties in cats and dogs are disclosed by Ghys et.al. (Arzneimittelforschung 1985;35(10): 1520-6), Masami et.al. (J. Vet. Med. Sci. 2003;65(10):1057-1061) and in dogs by Uchida et.al. (J. Pharm. Pharmacol. 1992 Jan;44(1):39-44). Matsuoka et.al. disclose the use of torasemide in the treatment of congestive heart failure or edema in cats and dogs (J Vet Med Sci. 2003 Oct;65(10)1057-61).

Torasemide is further used in humans to treat edema associated with congestive heart failure or to treat high blood pressure. Further uses of torasemide in the treatment of heart failure, cirrhosis, pulmonary hypertension, chronic kidney disease and acute kidney disease in humans are reviewed by Wargo and Banta in The Annals of Pharmacotherapy, 2009 November, Volume 43.

None of the documents disclose the use of torasemide in the alleviation or treatment of one or more symptoms caused by one or more conditions or diseases selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof. Patients suffering from these disease can suffer greatly from symptoms caused by one or more of these diseases. Up till now it was not recognized that torasemide can be used in the treatment or alleviation of certain symptoms more specifically symptoms caused by one or more conditions or diseases selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof.

Hence it is an object of the invention to provide for the alleviation or treatment of one or more symptoms caused by one or more conditions or diseases selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof.

This object is achieved by invention according to the preamble by the use of torasemide in the alleviation or treatment of symptoms caused by one or more conditions or diseases selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof.

Torasemide is available on the market as Torasemide by Cosma SpA IT 24040 Ciserano (Bergamo) and by S.I.M.S. S.R.L. IT 50066 Reggello. Torasemide has several advantages, for example it has a longer duration of action, a higher potency, a stronger effect of action, less side effects and the patient develops no resistance if compared to current diuretics like furosemide.

With patient(s) is meant one or more mammals and preferably dog and/or cat suffering from dilated cardiomyopathy, acquired or naturally occurring valvular disease, hypertrophic cardiomyopathy, hypertrophic obstructive cardiomyopathy, restrictive cardiomyopathy and unclassified cardiomyopathy or combinations thereof.

The present inventors have found that the use of a composition comprising torasemide as a veterinary medicament can alleviate or treat symptoms caused by one or more conditions or diseases, selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof. This greatly reduces the suffering of patients and hence improves the quality of life of the patient.

The present inventors have found that if the disease is one or more chosen from the group consisting of dilated cardiomyopathy, acquired or naturally occurring valvular disease, and unclassified cardiomyopathies and combinations thereof, the use of torasemide in the alleviation or treatment of symptoms caused by the diseases is especially effective. Hence the suffering of patients caused by said diseases is reduced and the quality of life of the patient improved.

In an embodiment of the present invention the composition further comprises at least one additional medicament, selected from the group, consisting of pimobendan (a benzimidazole-pyridazinone derivative), one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

In an embodiment of the present invention the composition is provided to a subject in combination with a separate dose of at least one additional medicament, selected from the group, consisting of pimobendan (a benzimidazole-pyridazinone derivative), one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

In an embodiment of the present invention the veterinary medicament is for cats and/or dogs.

In an embodiment of the present invention the veterinary medicament is for a cat and the additional medicament is selected from the group, consisting of pimobendan (a benzimidazole-pyridazinone derivative), one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

In an embodiment of the present invention the veterinary medicament is for a dog and the composition comprises or is provided to a subject in combination with pimobendan.

Preferably one or more of the crystalline forms of pimobendan as described in NL1037569 is used. Suitable daily dosages of pimobendan are maximally 0.6 mg /kg body weight of the subject. The solubility characteristics of said crystalline forms are such that adding an organic acid or an anhydride thereof is not needed for ensuring a satisfactory dissolution rate and therefore satisfactory resorption of pimobendan.

In an embodiment of the present invention the veterinary medicament is for the alleviation or treatment of symptoms caused by a condition or disease selected from the group consisting of dilated cardiomyopathy, acquired or naturally occurring valvular disease, or unclassified cardiomyopathies and where an augmentation of cardiac output is possible.

In an embodiment of the present invention the medicament is provided to the subject in a dosage of at the most 5mg/kg body weight of the subject, and preferably 0.025 to 0.29 mg/kg body weight of the subject or a dosage of 0.31 to 5 mg/kg body weight of the subject. Said dosage is preferably administered once daily. Within these ranges the present inventors observed a good effect of the present invention.

In an embodiment of the present invention the composition is administered orally, sublingually, intramuscularly, subcutaneously, intravenously or transdermally, preferably the composition according to the present invention is administered orally.

In an embodiment of the present invention the composition is a formulation for parenteral administration and preferably comprises torasemide, preservative(s), isotonic agent(s), buffering agent(s) and solvent(s). Preferably torasemide is present as anhydrous torasemide.

In an embodiment of the present invention the composition is in tablet form and comprises torasemide, one or more diluent(s) and/or filler(s), disintegrant(s), lubricant(s) and colorant(s) and preferably one or more flavourants. Preferably torasemide is present as anhydrous torasemide.

In an embodiment of the present invention the composition is a palatable composition. The acceptance of the composition by the subjects is improved and force-feeding of subjects can be avoided with the palatable composition. This leads to a high compliance of the subjects to be treated.

The present invention further relates to a kit comprising a composition comprising torasemide for use according to the present invention and further comprising a package leaflet or user instruction including information for the use according to the present invention.

The present invention further relates to a method for alleviating or treating symptoms in an animal caused by one or more conditions or diseases, selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof comprising the step of administering to the animal a composition comprising torasemide.

### Example:

As an example for the use of the composition according to the present invention a formulation for parenteral administration comprises the following:
Torasemide (anhydrous)
Preservative(s)
Isotonic agent(s)
Buffering agent(s)
Solvent(s)

As an example for the use of the composition according to the present invention a formulation for a tablet for oral administration comprises the following ingredients: Torasemide (anhydrous)
Diluent(s) / Filler(s)
Disintegrant(s)
Lubricant(s)
Colorant(s)
Flavour(s)

## Claims

1. Composition comprising torasemide for use as a veterinary medicament for the alleviation or treatment of symptoms caused by one or more conditions or diseases, selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof.

2. Composition according to claim 1, **characterized in that** the disease is one or more selected from the group consisting of dilated cardiomyopathy, acquired or naturally occurring valvular disease, and unclassified cardiomyopathies and combinations thereof.

3. Composition according to claim 1 or 2, **characterized in that** the composition further comprises at least one additional medicament, selected from the group, consisting of pimobendan (a benzimidazole-pyridazinone derivative), one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

4. Composition according to claim 1 or 2 , **characterized in that** the composition is provided to a subject in combination with a separate dose of at least one additional medicament, selected from the group, consisting of pimobendan (a benzimidazole-pyridazinone derivative), one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

5. Composition according to any one or more of the preceding claims, **characterized in that** the veterinary medicament is for mammals, and preferably cats and/or dogs.

6. Composition according to claim 5, **characterized in that** the veterinary medicament is for a cat and the additional medicament is selected from the group, consisting of pimobendan, one or more ACE inhibitors (e.g. benazepril, enalapril and ramipril), one or more β-blockers (e.g. propanolol, atenolol), one or more cardiac glycosides (e.g. digoxin), one or more aldosteron antagonists (e.g. spironolactone), one or more calcium channel blockers (e.g. amlodipine), one or more bronchodilatators and combinations thereof.

7. Composition according to any one or more of claims 3-5, **characterized in that** the veterinary medicament is for a cat and/or a dog and the composition comprises pimobendan or is provided to a subject in combination with pimobendan.

8. Composition according to claim 5, **characterized in that** the veterinary medicament is for the alleviation or treatment of symptoms caused by a condition or disease selected from the group consisting of dilated cardiomyopathy, acquired or naturally occurring valvular disease, or unclassified cardiomyopathies and where an augmentation of cardiac output is possible.

9. Composition according to any one of claims 1-8 **characterized in that** the medicament is provided to the subject in a dosage of 0.025 to 0.29 mg/kg or a dosage of 0.31 to 5 mg/kg once daily.

10. Composition according to any one or more of the preceding claims, **characterized in that** the composition is a palatable composition.

11. A kit comprising a composition according to one or more of the previous claims and further comprising a package leaflet or user instruction including information for the use according to any one or more of the preceding claims.

12. Composition comprising torasemide for use as a veterinary medicament.

13. Method for alleviating or treating symptoms in an animal caused by one or more conditions or diseases, selected from the group consisting of dilated cardiomyopathy, acquired or naturally occurring valvular disease, hypertrophic cardiomyopathy, hypertrophic obstructive cardiomyopathy, restrictive cardiomyopathy and unclassified cardiomyopathy and combinations thereof comprising the step of administering to the animal a composition comprising torasemide.
